# EUROPEAN PATENT SPECIFICATION

(11) **EP 3 790 488 B1**
(45) Date of publication and mention of the grant of the patent: **25.06.2025**
(21) Application number: 19800741.1
(22) Date of filing: 07.05.2019
(51) Int. Cl.: A61B 18/14, A61B 17/34, A61B 90/00, A61B 5/00, A61B 18/00

(54) **APPARATUS FOR PUNCTURING TISSUE**
VORRICHTUNG ZUR PUNKTIERUNG VON GEWEBE
APPAREIL POUR PERFORER UN TISSU

(30) Priority: 08.05.2018 US 201862668396 P
(43) Date of publication of application: 17.03.2021
(60) Divisional application: 25168296.9
(73) Proprietor: Boston Scientific Medical Device Limited, Galway H91 Y868 (IE)
(72) Inventor: URBANSKI, John Paul, Missisauga, Ontario L4W 5S4 (CA); MILLER, Brock, Missisauga, Ontario L4W 5S4 (CA); GRAVETT, Matthew, Milton, Ontario L9T 8L6 (CA); ABOU-MARIE, Rund, Missisauga, Ontario L4W 5S4 (CA); LUK, Maria, Missisauga, Ontario L4W 5S4 (CA); KAMAL, Ahmad, Missisauga, Ontario L4W 5S4 (CA)
(74) Representative: Pfenning, Meinig & Partner mbB
(86) International application number: PCT/IB2019/053751
(87) International publication number: WO 2019/215621

(56) References cited:
- WO-A1-2015/019132
- WO-A1-2017/042743
- JP-B2- 6 301 926
- US-A1- 2004 133 113
- US-A1- 2014 228 841
- US-A1- 2015 157 353
- US-A1- 2017 172 653
- US-A1- 2017 266 433

## Description

### TECHNICAL FIELD

The disclosure relates to systems of delivering energy to tissue. More specifically, it relates to systems for delivering energy using electrosurgical devices to puncture tissue. US 2014/228841 A1 discloses devices and methods for providing access to the pericardial cavity while reducing risk of myocardial damage. One method includes advancing a puncture device towards a heart, the puncture device including an energy delivery device; measuring an electrical impedance at the energy delivery device; delivering energy from the energy delivery device to at least partially puncture a pericardium; and repeating one or more of the above steps, if necessary, until the energy delivery device is located at least partially within the pericardial cavity. Some embodiments of the method include using supplemental means of monitoring, including measuring voltage to plot an ECG, medical imaging and using contrast fluid, using tactile feedback, and aspirating fluid.
WO 2015/019132 A1 discloses medical devices and associated methods for facilitating efficient and repeatable puncture of a tissue site while allowing vascular access from various access sites of a patient's body. Disclosed medical devices include dilators and wires usable alone or in combination and configured to facilitate tissue access and puncture at various anatomical locations from desired access sites. The medical devices each include one or more sections having sufficient flexibility for accessing the tissue site from the access site while retaining sufficient stiffness to perform one or more additional functions. US 2017/266433 A1 discloses guidewires and methods for transmitting electrical stimuli to a heart and for guiding and supporting the delivery of elongate treatment devices within the heart. A guidewire can comprise an elongate body, including first and second elongate conductors, and at least first and second electrodes. A distal end portion of the elongate body can include a preformed bias shape, such as a pigtail-shaped region, on which the first and second electrodes can be located. The preformed bias shape can optionally be non-coplanar relative to an intermediate portion of the elongate body. The first and second elongate conductors can be formed of a single structure or two or more electrically connected structures. The conductors can extend from proximal end portions to distal end portions that electrically connect to the first and second electrodes. A core wire can extend the length of the elongate body, can at least partially form the first conductor, and can be at least partially surrounded by the second conductor.

### SUMMARY

The invention is defined in independent claim 1. Certain optional features of the invention are defined in the dependent claims. The methods described herein do not form part of the invention. The safety of a procedure for puncturing a target tissue with a puncture device can be increased using a method of confirming the position of the tip of the puncture device relative to the target tissue. The method uses an elongate device (e.g. a radiofrequency (RF) guidewire) having a tip electrode, which is configured for collecting electrograms (EGMs) and for delivering electrical energy for puncturing the tissue, and the method including collecting EGMs to indirectly measure and monitor the pressure applied against the target tissue by the elongate device to thereby indicate tip location with respect to the target tissue. In some embodiments the target tissue is on a pericardium and the method includes collecting epicardial electrograms (EGMs) to measure and monitor the pressure applied against the pericardial target tissue site. In other embodiments, the tissue is some part of a body other than a pericardium, for example, a septum of a heart.

A method of confirming a position of a tip of a puncture device relative to a target tissue which includes using an elongate puncture device having a tip electrode that is configured for collecting EGMs and for delivering energy for puncturing tissue is disclosed for putting the present invention into a context for facilitating the understanding. The method comprises a step of collecting EGMs with the tip electrode to measure and monitor a pressure applied against the target tissue by the tip electrode of the elongate puncture device.

In some embodiments of the first broad aspect, the target tissue is a pericardium. In some other embodiments, the target tissue is a septum of a heart, and in some such embodiments, the target tissue is an atrial septum. In some examples of the first broad embodiment, the elongate puncture device delivers radiofrequency energy, and in some such examples the elongate puncture device is a radiofrequency guidewire, or a radiofrequency stylet, or a radiofrequency trocar. In some examples, the EGMs are collected from a pericardium, and in some other examples, the EGMs are collected from a septum of a heart. In some embodiments, the elongate puncture device applies pressure against pericardial tissue, and in other embodiments, the elongate puncture device applies pressure against a septum of a heart.

In a further example, a method of puncturing a target tissue which includes using an elongate puncture device having a tip electrode that is configured for collecting EGMs and for delivering energy for puncturing the target tissue, the method comprising collecting EGMs to measure and monitor a pressure applied against the target tissue by the elongate puncture device, thereby confirming a position of the tip electrode of the elongate puncture device relative to the target tissue is disclosed.

In some examples, the target tissue is a pericardium. In other embodiments, the target tissue is a septum of a heart, and in some such embodiments, the target tissue is an atrial septum. In some examples of the second broad embodiment, the elongate puncture device delivers radiofrequency energy, and in some such examples, the elongate puncture device is a radiofrequency guidewire. In some examples of the second broad aspect, EGMs are collected from a pericardium, and in some other examples, EGMs are collected from a septum of a heart. In some embodiments of the second broad aspect, the elongate puncture device applies pressure against pericardial tissue, and in other embodiments, the elongate puncture device applies pressure against a septum of a heart.

In a further example, a method of gaining access to a pericardial cavity comprises the steps of: (a) introducing a blunt tipped stylet into an introducer; (b) advancing the blunt tipped stylet and the introducer, in combination, through adipose tissue 2 0 towards a tissue of the pericardium; (c) removing the stylet from the introducer; (d) installing an elongate puncture device, which is flexible, in the introducer; (e) advancing the tip of the elongate puncture device to be distal of the introducer under fluoroscopy whereby an electrode of the elongate puncture device is in contact with a pericardium without any significant force being exerted; (f) monitoring an EGM based on the electrode of the elongate puncture device which is in contact with the pericardium to confirm there is a low ST segment; (g) exerting force with the introducer and the electrode of the guidewire to tent the pericardium; (h) monitoring the EGM to confirm the wave is higher than the wave of step (f); (i) delivering energy through the electrode to the tissue of the pericardium; and G) monitoring the EGM to confirm the ST segment is higher than the waves of steps (f) and (h).

In typical examples, step (i) includes delivering a short pulse of high voltage AC. In some such embodiments, the short pulse is 1/3 second. Typical embodiments further comprise a step (k) of advancing the distal portion of the introducer into the pericardial cavity and monitoring the EGM.

In some examples, steps (e) and (f) are performed simultaneously. In some examples, steps (g) and (h) are performed simultaneously. In some examples, steps (i) and G) are performed simultaneously.

Some examples further comprise a step (1) of delivering a contrast media. Some embodiments further comprise a step (m) of withdrawing fluid from the pericardial space. Some embodiments further comprise a step (n) of advancing the elongate puncture device into the pericardial cavity. Some embodiments further comprise a step (o) of confirming access by wrapping the guidewire around the heart at least once and visualizing under fluoroscopy.

In a further example, a method of puncturing a target tissue to gain access to the pericardial cavity comprises the steps of: (1) connecting an electrically insulated wire having one distal pole to a recording system that has an electrocardiographic reference to the patient; (2) puncturing the patient's skin via the subxiphoid, parasternal intercostal or apical approach with a rigid introducer and an engaged stylet; (3) advancing the rigid introducer and the stylet through various dermal layers and toward the pericardial sac of the heart under the guidance of fluoroscopy and tactile feedback to position the tip of the introducer near the pericardial sac; (4) removing the stylet while a blunt radial tip of the introducer is docking with the pericardial sac, and installing the elongate puncture device in the introducer; (5) advancing the elongate puncture device while the introducer is contacting the outside of the pericardial sac; and (6) delivering energy to the pericardial sac through the electrode at the tip of Elongate puncture device.

In typical examples, step (5) includes recording local epicardial EGMs from the electrode tip of the elongate puncture device. In some such embodiments, step (5) further comprises monitoring the EGM to confirm the amplitude of the waveforms increases with higher mechanical force against the heart.

In some examples, step (6) comprises the energy being delivered as at least one pulse. In some other examplesct, step (6) comprises the energy being delivered as a series of pulses, starting with a low pressure level against the pericardial sac in the first iteration to avoid overshoot, and the amount of pressure exerted on the pericardial sac is increased with each iteration while maintaining the same pulse time and energy level. In some examples step (6) comprises checking if the pericardial sac has been punctured and access to the pericardial cavity has been gained.

Typical embodiments of the fourth broad aspect further comprise a step (7) of monitoring the electrical activity of the epicardial surface using the electrode on the distal tip of the elongate puncture device (after RF puncture of the pericardial sac provides access to the pericardial cavity). Some embodiments further comprise a step (8) of deploying the elongate puncture device further into the pericardial space to relax pressure at the tip and cause a change in the EGM trace. Some embodiments further comprise a step (9) of deploying the guidewire for tracking along the epicardial surface of the heart while collecting local epicardial EGM. Some embodiments further comprise a step of advancing a sheath and dilator over the elongate puncture device.

In a further example, a method of confirming a position of a tip of an elongate puncture device relative to an introducer wherein the elongate puncture device has a proximal marker which is visible to a naked eye and a distal tip marker which is visible under an imaging system, and the introducer has distal end marker which is visible under the imaging system. The method includes the steps of: (1) positioning the elongate puncture device relative to a proximal end of the introducer using the proximal marker without an imaging system in a macro-positioning step; (2) turning on the imaging system; and (3) positioning a distal tip of the elongate puncture device relative to an end of introducer by viewing the distal tip marker and distal end marker using the imaging system in a micro-positioning step. In some such examples, the imaging system is a fluoroscopy system and the distal tip marker and distal end marker are visible under fluoroscopy.

In some examples, the elongate puncture device comprises a radiofrequency guidewire.

In a first broad aspect, embodiments of the present invention include an elongate puncture device comprising a mandrel which is electrically conductive and covered by a clear layer of insulation, the clear layer stopping short of a distal end of the mandrel such that such that the distal end of the mandrel is electrically exposed to define a distal tip electrode, a portion of the mandrel being surrounded by a visible marker, the visible marker being covered by the clear layer, wherein the portions of the elongate puncture device at and adjacent the visible marker have a constant outer diameter.

In typical embodiments of the first broad aspect, the mandrel is surrounded by an oxide coating which is covered by the clear layer of insulation, wherein for at least one portion of the mandrel the oxide coating has been removed such that said at least one portion defines at least one visible marker.

In some embodiments of the first broad aspect, the at least one visible marker comprises at least one portion of the mandrel wherein the oxide coating is in contact with the mandrel. In some such embodiments, the oxide coating is comprised of a layer of titanium dioxide. Embodiments include the visible marker may be a proximal marker, an intermediate marker, or a distal marker. In typical embodiments, the clear layer comprises a heat-shrink layer, wherein in some such embodiments the heat-shrink layer comprises a polytetrafluoroethylene material.

In some embodiments of the first broad aspect, the mandrel is comprised of a nitinol. In some other embodiments, the mandrel is comprised of a stainless steel. In typical embodiments, the elongate puncture device is flexible.

In typical embodiments of the first broad aspect, the mandrel is electrically conductive, and a proximal end portion of the mandrel is uninsulated and operable for connecting to a power supply such that the distal tip electrode is in electrical communication with the power supply, and energy can be delivered through the distal tip electrode to tissue, and the distal tip electrode enables recording epicardial EGM. Such embodiments typically include a shaft of the elongate puncture device being electrically insulated to reduce noise from any collected local EGM. In some embodiments, the distal tip electrode has a hemispherical dome with an outer diameter> .027" (0.686 mm) and a surface area of about 1.8 to about 2.4 mm2.

Typical embodiments of the first broad aspect further comprise a distal end portion 2 0 which has a J-profile. Such embodiments typically further comprise a radiopaque coil which extends around a curve of the distal end portion which has a J-profile. In such embodiments, an end of the radiopaque coil can be used as a distal tip marker. In some examples, the radiopaque coil has echogenic properties when using ultrasound to enable visualization of the guidewire tip.

In some embodiments of the first broad, the elongate puncture device comprises a radiofrequency guidewire.

In a second broad aspect, embodiments of the present invention include an introducer for use with an elongate puncture device, the introducer comprising an introducer shaft connected to a hub, the introducer shaft comprising an internal metal tube which is interposed between two layers of insulation with both ends of the two layers being joined together to bond the layers. In typical examples, the two layers of insulation are comprised of high-density polyethylene. In some examples, the hub further comprise a male side port for connecting to tubing and a receiving opening for receiving a stylet or a wire whereby the hub is operable to be simultaneously attached to source of fluid while a stylet or a wire is inserted into the hub.

In an example not forming part of the claimed invention, a kit is disclosed comprising an introducer and an elongate puncture device, the elongate puncture device including a distal tip electrode which enables recording epicardial EGM and a shaft of the elongate puncture device is electrically insulated to reduce noise in any collected local EGM; and the introducer comprising an introducer shaft which connected to a hub, the introducer shaft comprising an internal metal tube which is interposed between two layers of electrical insulation with both ends of the two layers being joined together to bond the layers to reduce noise in any EGM collected by the elongate puncture device. In typical examples, the introducer and the elongate puncture device are configured such that the introducer has the ability to deliver and withdraw fluid while the elongate puncture device is inserted therethrough. In some examples, the radial gap between an inner diameter of a tip of the introducer and outer diameter of the elongate puncture device is> 0.025 mm (.001"). In some such examples, the kit is operable to provide contrast flow> 15ml/min at 69 Kilo Pascal (10 PSI). In some examples, the inner diameter of a tip of the introducer is 0.978 mm (.0385 inches) +/- 15 0.013 mm (.0005 inches), and the maximum outer diameter of the elongate puncture device is 0.889 mm (.0350 inches) +/- 0.013 mm (.0005 inches), whereby the minimum gap with this geometry is 0.032 mm (.00125").

Some examples further comprise a stylet wherein a cross section of the stylet varies along the length of the stylet, tapering down towards a distal tip of the stylet to allow a flow of a fluid through a tip of the introducer when the stylet is installed. Some embodiments further comprise a stylet wherein the stylet is electrically insulated and operable to be connected to a recording system to facilitate collecting EGM information to help in positioning a tip of the introducer when approaching the heart.

Some examples further comprise markers for positioning the elongate puncture device relative to the introducer wherein the elongate puncture device has a proximal marker which is visible to a naked eye and a distal tip marker which is visible under an imaging system, and the introducer has distal end marker which is visible under the imaging system. In typical embodiments, the distal tip marker and distal end marker are visible under fluoroscopy.

In some examples, a tip of the elongate puncture device extends distal of a shaft of the introducer shaft when the proximal marker of the elongate puncture device is positioned at a proximal end of a hub of the introducer. In some other examples, a tip of the elongate puncture device lines up with a distal end of a shaft of the introducer shaft when the proximal marker of the elongate puncture device is positioned at a proximal end of a hub of the introducer.

In some examples, the elongate puncture device comprises a radiofrequency guidewire.

### BRIEF DESCRIPTION OF THE DRAWINGS

In order that the invention may be readily understood, embodiments of the invention are illustrated by way of examples in the accompanying drawings, in which:
Fig. 1 is an illustration of a radiofrequency (RF) guidewire in accordance with an embodiment of the present invention;
Fig. 2 is a cross sectional view of the embodiment of Fig. 1;
Fig. 3 is an illustration of a detail of Fig. 2;
Fig. 4 is an illustration of a stylet in accordance with an embodiment of the present invention;
Fig. 5 is a cross sectional view of the embodiment of Fig. 4;
Fig. 6 is an illustration of detail A of Fig. 4;
Fig. 7 is an perspective view of an introducer with a stylet contained therein in accordance with an embodiment of the present invention;
Fig. 8 is a side view of the embodiment of Fig. 7;
Fig. 9 is an end view of the embodiment of Fig. 7;
Fig. 10 is a top view of the embodiment of Fig. 7;
Fig. 11 is a cross sectional view of the embodiment of Fig. 8;
Fig. 12 is an illustration of detail A of Fig. 11;
Fig. 13 is an illustration of apparatus in accordance with an embodiment of the present invention;
Fig. 14 is an illustration of the distal end of an introducer with a with an elongate puncture device contained therein in accordance with an embodiment of the present invention;
Fig. 15 is an illustration of the embodiment of Fig. 14 with the elongate puncture device extended to form a J-tip;
Fig. 16 is an illustration of the proximal end of the hub of Fig. 13 with the elongate puncture device inserted therein;
Fig. 17 is a diagrammatic cross sectional view of an introducer with an elongate puncture device installed therein in accordance with an embodiment of the present invention;
Fig. 18 is an illustration of the layers surrounding a heart;
Fig. 19 is an illustration of EGM waves showing a sequence of raised ST segments;
Fig. 20-1, and Figs. 20A to Fig. 20F illustrate a method in accordance with an embodiment of the present invention;
Figs. 21A and 21B illustrate possible problem scenarios;
Fig. 22A is an illustration of different maker configurations of an elongate puncture device;
Fig. 22B is a cross section of Fig. 22A;
Fig. 23A is an illustration of fluid flow from a radial gap at a distal end if an introducer;
Fig. 23B is an illustration of an introducer with a side port for fluid flow;
Fig. 24 is an illustration different stylet embodiments;
Fig. 25 is an illustration of an alternative embodiment of a mandrel;
Fig. 26 is an illustration of an introducer hub with a tip straightener;
Fig. 27 is an enlarged view of a portion of Fig. 26;
Fig. 28 is an illustration of alternative embodiments of the introducer distal tip;
Fig. 29A is an illustration of an introducer with a flexible tip and an inserted stylet;
Fig. 29B is an illustration of the introducer of Fig. 29A with the stylet removed; and
Figs. 30A to 30C illustrate the steps of a method of advancing an elongate puncture device through an introducer shaft.

### DETAILED DESCRIPTION

Minimally invasive catheterization of the pericardial space is required for diagnostic and treatment of a variety of arrhythmias. Although epicardial ablation is a preferred route in many situations (for ventricular tachycardias, for instance), physicians may resort to common endocardial ablation due to uncertainties involved in access and high clinical complication rate. The current standard procedure for epicardial access is facilitated by using a 17 Ga Tuohy needle that percutaneously punctures the pericardial sac via the subxiphoid or parasternal intercostal or apical approach. This puncture is typically performed under fluoroscopic guidance using a combination of anterior-posterior and lateral views. Radiopaque contrast agents are periodically injected to provide positive feedback to the user on tip position, and whether or not the needle tip has successfully broached the pericardial sack. With this approach there is the possibility of lacerating the epicardium, cardiac vessels or surrounding soft tissue structures. There is also the risk of inadvertent entry into the ventricle that can lead to an effusion and perhaps tamponade. Any new devices or methods to improve the safety and predictability to confirm targeted tissue site and reduce chance of complications would be beneficial.

The inventors have come up with a unique and heretofore undiscovered solution of improving the safety of a procedure for puncturing a target tissue with a puncture device, which includes using a method of confirming the position of the tip of the puncture device relative to the target tissue. The method, not forming part of the present invention, uses an elongate device (e.g. a radiofrequency (RF) guidewire) having a tip electrode which is configured for collecting local electrograms (EGMs) and for delivering energy for puncturing the tissue, with the method including collecting EGMs to indicate tip location with respect to the target tissue. The elongate device doesn't specifically measure the EGM, but passes on the signal to EGM signal processing equipment. In some embodiments the target tissue is on a pericardium and the method includes collecting local epicardial electrograms (EGMs) to indirectly measure and monitor the pressure applied against the pericardial target tissue site. In other embodiments, the tissue is a septum of a heart and the method includes collecting intracardiac electrograms (EGMs) to indirectly measure and monitor the pressure applied against the septum. Typical embodiments include collecting an EGM signal during tenting of the target tissue.

One embodiment is for a guidewire that is operable to provide tip information back to the user on confirming location of the tip of the device in reference to the targeted tissue. The invention uses local epicardial electrograms (EGM) to aid in confirming location of the guidewire tip when approaching the heart, docking with the targeted tissue before and after puncturing the sac.

With specific reference now to the drawings in detail, it is stressed that the particulars shown are by way of example and for purposes of illustrative discussion of certain embodiments of the present invention only. It is to be understood that the invention is not limited in its application to the details of construction and the arrangement of the components set forth in the following description or illustrated in the drawings. The invention is capable of other embodiments or of being practiced or carried out in various ways. Also, it is to be understood that the phraseology and terminology employed herein is for the purpose of description and should not be regarded as limiting.

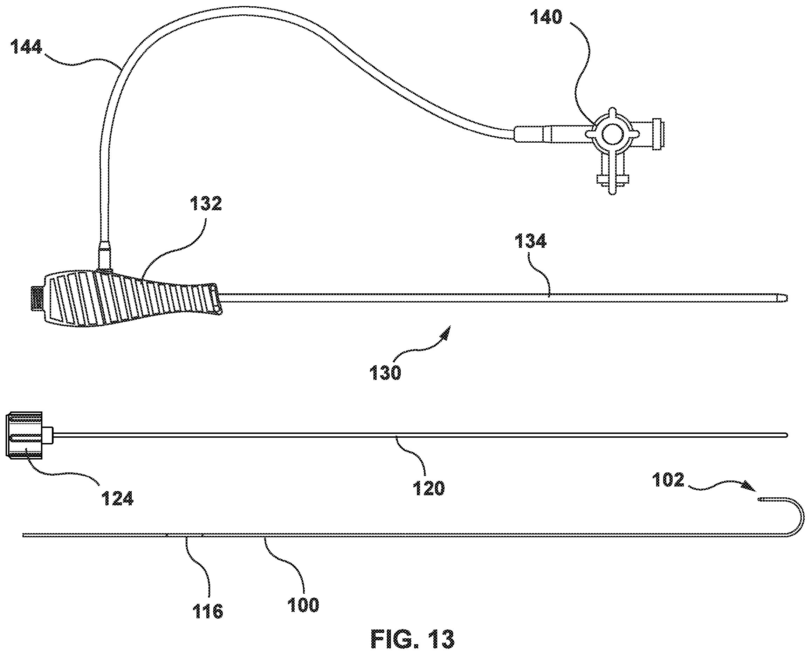

An example a system (or a kit) of apparatus suitable for performing the methods disclosed herein is shown in Fig. 13. The apparatus includes elongate puncture device 100, stylet 120, and introducer 130. While this disclosure uses the term "elongate puncture device" with regards to elongate puncture device 100 for explanatory purposes, elongate puncture device 100 is, in general, an elongate member capable of delivering energy to tissue through a distal end electrode. Elongate puncture device 100 is flexible in some embodiments and stiff in other embodiments. Examples of flexible embodiments include wires capable of delivering electrical energy, such as RF guidewires. Examples of stiff embodiments include needles operable for gaining access to pericardial cavities (some of which have a form and feel which resembles a 17Ga-20Ga Tuohy needle) or for gaining transseptal access (some of which have a form and feel which resembles a Brockenbrough needle). Also, typical embodiments of elongate puncture device 100 are operable to deliver electrical energy at frequencies other than radiofrequency. The energy frequency used in a given procedure is typically determined by the user selecting a frequency from the range of frequencies available with the generator being used in the procedure.

Introducer 130 of Fig. 13 is connected to valve 140 by tubing 144. In the illustrated 20 embodiment, valve 140 is a 3-way stopcock valve. Alternative embodiments have other valve configurations that are operable to deliver and withdraw fluid. The apparatus of Fig. 13 may be used for a variety of tissue puncture procedures, for example, pericardial puncture procedures or transseptal puncture procedures. The main components of the apparatus shown in Fig. 13 are described in greater detail below.

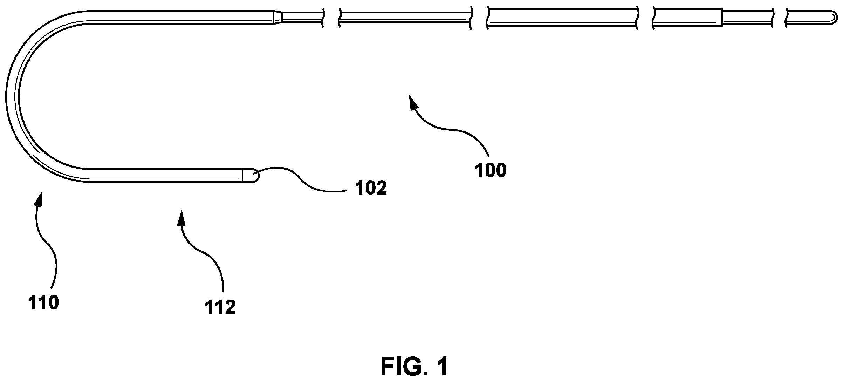

Referring to Fig. 1, an embodiment of elongate puncture device 100 comprises a distal end portion 110 which has a J-profile and a straight portion 112 at the distal end thereof. The elongate puncture device assumes the J-shape when not constrained. The J-profile of the distal end portion 110 helps minimize tissue trauma when tracking the distal tip across the heart's surface. The distal end portion 110 could also be a 'P' or a straight profile. Some embodiments have a distal tip angle from 0 to 35 degrees from the longitudinal axis of the main shaft. The idea is to keep the distal tip's straight portion 112 tucked in as it tracks around the heart to prevent it from swinging out and impinging adjacent soft tissue structures. The distal curve should also be in-plane with the wire body for predictable advancement of the device. One embodiment has a J-profile with an outside diameter of 10 - 12 mm. The straight portion 112 of the distal tip allows for alignment and orientation of the elongate puncture device tip to the introducer to facilitate RF puncture. One embodiment has a length of straight active tip which is between 6 - 9 mm in length. This length is required to allow the user to control alignment of the elongate puncture device by hand. Distal tip straight portion 112 has adequate body length to allow for alignment, positioning of the tip with respect to the introducer.

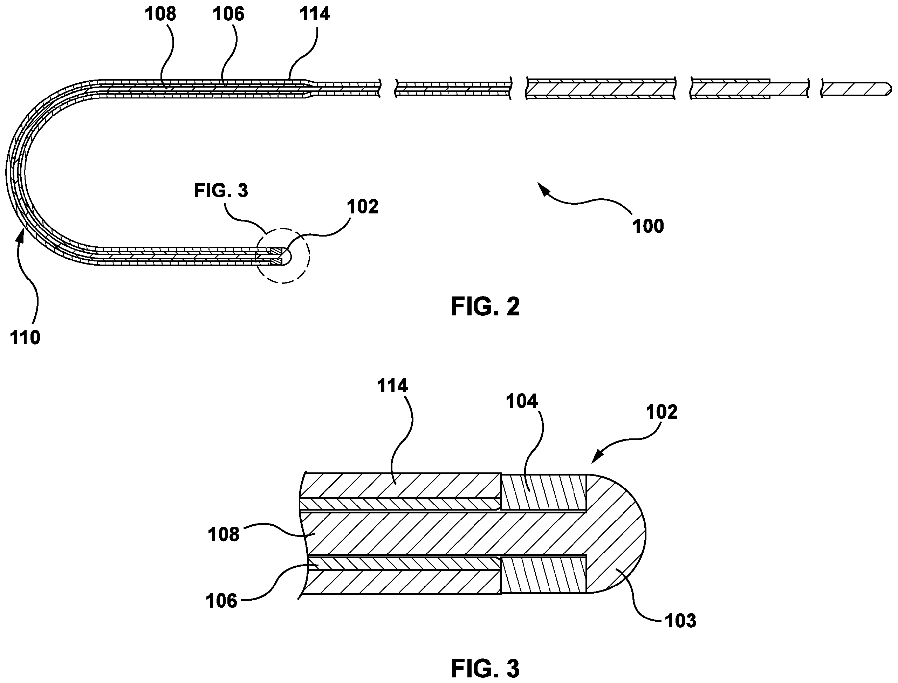

Electrode 102 is at the end of straight portion 112. The guidewire has a blunt electrode at the tip to safely dock with tissue and thereby facilitate directly delivering RF energy to the targeted tissue which the electrode is in contact with. Typically the electrode 102 has a dome profile which minimizes the opportunity for premature mechanical puncture, and allows the elongate puncture device to dock with the target tissue at various angles. Embodiments of electrode dome 103 may have a shape which is hemispherical, ellipsoid, or a paraboloid. In some embodiments, electrode dome 103 retains radiopaque marker 104. In alternative embodiments, other orientations and materials are used to secure radiopaque material to the distal end of elongate puncture device 100. Referring to Fig. 2, the proximal end portion of elongate puncture device 100 (I.e. the end which is at the end opposite to the end of the wire having electrode 102) is uninsulated and is operable for connecting to a power supply or generator. In some embodiments, the proximal end electrically exposed mandrel 108 is about 7.5 +/- 2.5 mm in length. In typical embodiments, the mandrel 108 is electrically conductive, and a proximal end portion of the mandrel 108 is uninsulated and operable for connecting to a power supply such that the distal tip electrode 102 is in electrical communication with the power supply, whereby energy can be delivered through the distal tip electrode 102 to tissue and the distal tip electrode enables recording epicardial EGM.

Elongate puncture device 100 of Fig 13 includes a proximal marker 116. Laser etching can be used to form proximal marker 116 so that it cannot be removed during use or sterilization. The use of proximal marker 116 is described below.

Some embodiments of elongate puncture device 100 comprises a mandrel 108 which is electrically conductive and covered by a clear layer of insulation 114 (clear heat-shrink 115), the clear layer stopping short of a distal end of the mandrel 108 such that such that the distal end of the mandrel 108 is electrically exposed (i.e. not covered) to define a distal tip electrode 102. A portion of mandrel 108 is surrounded by a visible marker 117, with the visible marker being covered by the clear layer, wherein the portions of the elongate puncture device at and adjacent the visible marker 117 have a constant outer diameter.

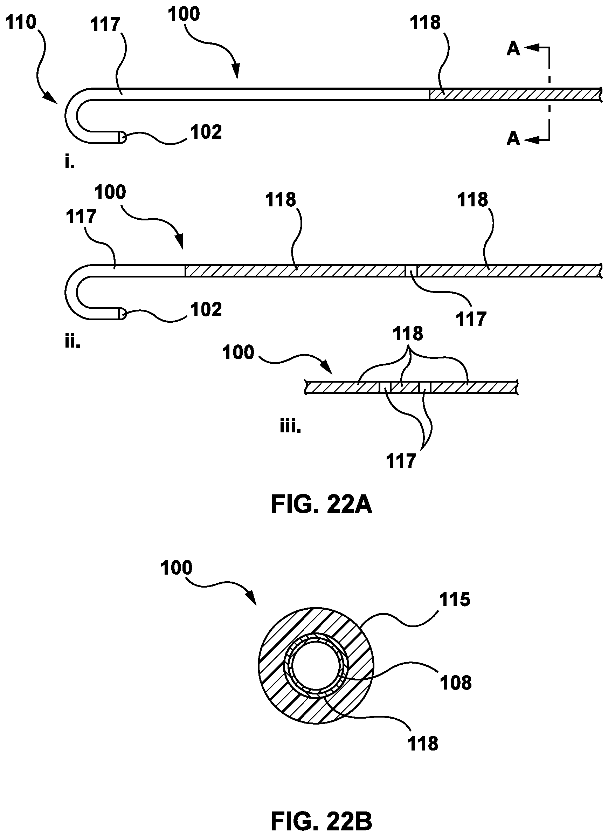

Some embodiments of elongate puncture device 100 include one or more marker 117 formed by mechanical grinding of an oxide coating of the wire created during heat treatment of the wire to fine-tune transformation temperatures. Marker 117 can be a proximal marker, an intermediate marker, or a distal marker. The formation of said markers is described making reference to Fig. 22A and 22B. Fig. 22b shows a cross-section of wire at point "A" of Fig. 22A after the wire is heat treated. Fig. 22B illustrates elongate puncture device 100 comprising a solid mandrel 100 surrounded by oxide coating 118 which is covered by clear heat-shrink 115 (a clear layer). In typical embodiments mandrel 108 is comprised of nitinol while in some alternative embodiments it is stainless steel. The typical oxide coating 118 on the wire is a titanium dioxide (Ti0(2)) oxide layer. This coating is typically stable and acts as a barrier against ion exchange. After the heat treatment, oxide coating 118 extends the full length of the wire. Typically a portion of the coating at the proximal end is removed to allow electrical connection with the over wire cable connectors and at least one other portion of the coating is removed to form a marker visible without imaging i.e. visible to an unaided eye. The oxide coating 118 can be removed by grinding the surface of the wire to the desired profile to thereby form a marker 117. Clear heat-shrink 115 typically comprises a Clear PTFE formed from an extruded tube that that is heat 15 shrunk onto the wire. The configuration of the markers of elongate puncture device 100 maintain a smaller consistent outer diameter (i.e. do not increase the outer diameter) while maintaining electrical integrity by including the markers under clear heat shrink 115. Alternative embodiments of heat-shrink 115 are comprised of a clear layer formed from alternative materials known to those skilled in the art. The elongate puncture device 100 is electrically insulated by the clear heat-shrink which allows a marker 117 to be visible. In some examples, the clear layer has a thickness ranging from about 0.086mm to 0.118mm.

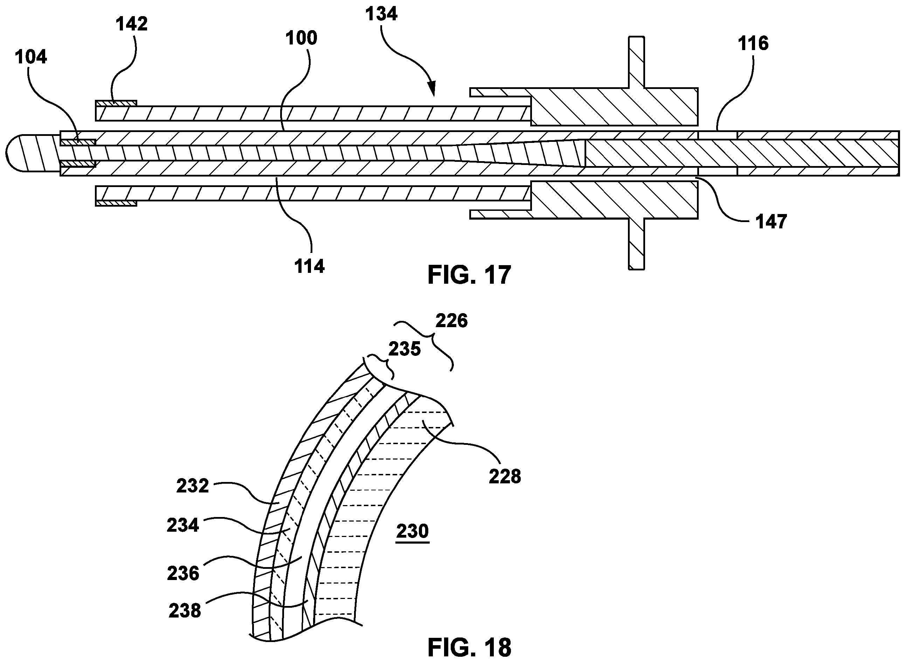

Fig. 22A shows different examples of marker 17. Fig. 22A-i shows a distal end marker 117. Fig. 22A-ii shows a distal end marker 117 and an intermediate marker 117. Fig. 22A-iii shows two intermediate markers 117. Proximal marker 116 of Fig. 17 could be formed by removing an oxide as described above and covering the wire with a clear layer. In some such embodiments, visible marker 117 comprises at least one portion of the mandrel wherein the oxide coating is applied directly to the mandrel i.e. in direct contact with the mandrel.

Fig. 3 illustrates detail C of Fig. 2. The embodiment of Fig. 3 includes a mandrel 108 (outer diameter 0.011 inches or 0.28 mm) with a radiopaque coil 106 (outer diameter 0.017 30 inches or 0.43 mm) covering a portion thereof. Insulation 114 (outer diameter 0.028 inches or 0.71 mm) covers the mandrel 108 and the radiopaque coil proximal of electrode 102 to enable delivery of electrical energy through electrode 102 (outer diameter 0.0275 inches or 0.70 mm, radius 0.01375 inches or 0.35 mm). In some embodiments the electrical insulation is comprised of heat shrink (e.g. polytetrafluoroethylene (PTFE)). Radiopaque coil 106 extends around the curve of distal end portion 110 (Fig 2). A radiopaque coil aids the physician in determining location of the distal tip of the elongate puncture device while using fluoroscopy. Physicians may reduce fluoroscopy intensity for long procedures reducing visibility of the guidewire. The coil can also improve echogenic properties when using ultrasound to visualize the guidewire tip. The coil can be 3 cm or longer. The coil also aids in visualizing the guidewire track around the cardiac silhouette. The coil aids to better visualize the distal tip's 'J' profile and how it interacts within the space. It can aid physicians in helping determine evidence of adhesions or anatomical abnormalities. The RO coil may be longer or shorter than 3 cm and may be constructed of various materials such as platinum, titanium, gold and tungsten. Some embodiments comprise a radiopaque coating around the mandrel. In some such embodiments, the wire is coated with a thin precious metal. Distal to radiopaque coil 106, electrode 102 includes a radiopaque marker 104 (outer diameter 0.0275 inches or 0.70 mm, inner diameter 0.011 inches or 0.28 mm, length 0.020 inches or 0.51 mm) which surrounds mandrel 108. Radiopaque marker 104 can also be referred to as a radiopaque (RO) band or a puck. A Radiopaque marker 104 helps visualize location of the tip under fluoroscopy. The RO band can help confirm if the tip of the elongate puncture device is protruding from the tip of the introducer 130. Embodiments of the RO band may be of varying lengths and constructed of various materials such as platinum, titanium, gold and tungsten. Elongate puncture device 100 is also visible using ultrasound imaging systems, radiopaque coil 106 being particularly echogenic.

Electrode 102 also includes an electrode dome 103 which may be formed by laser welding. In typical embodiments the mandrel of the wire, mandrel 108, is comprised of a shape memory material (e.g. nitinol) allowing it the ability to be kink resistant. The guidewire, when introduced into the pericardial space, may undergo sharp deflections. The flexible mandrel prevents any kinking, mitigating the possibility of getting the guidewire trapped in the body. In some alternative embodiments, mandrel 108 is constructed of stainless steel, which is less kink resistant. In other alternative embodiments, the mandrel is made of other super elastic materials with varying dimensions and cross-sections. In some embodiments, radiopaque coil 106 is comprised of tungsten. Some typical embodiments include radiopaque marker 104 being comprised of a mixture of platinum and iridium (e.g. 10% iridium), and electrode dome 103 being comprised of nitinol which has been dome welded. The distal tip region is floppy (i.e. not rigid) to minimize tissue trauma when tracking across the hearts surface. To achieve this, the mandrel at the distal end portion reduces in diameter necks from .025" (0.64 mm) down to .006" (0.15 mm) over a length of 25 cm. The tip will deflect creating a secondary bumper to that of the 'J' tip profile.

Elongate puncture device 100 has a length of about 150 to about 180 cm to ensure the guidewire can be deployed into the pericardial space and wrap around 1 to 2 times around the heart to define the cardiac silhouette under fluoroscopy. Alternative embodiments of the wire have a smaller or larger length to accommodate varying patient sizes and BMis e.g. lengths of 5 120 to about 180 cm. The elongate puncture device 100 typically has lubricous coating to ensure the guidewire tracks smoothly around the heart within the pericardial space.

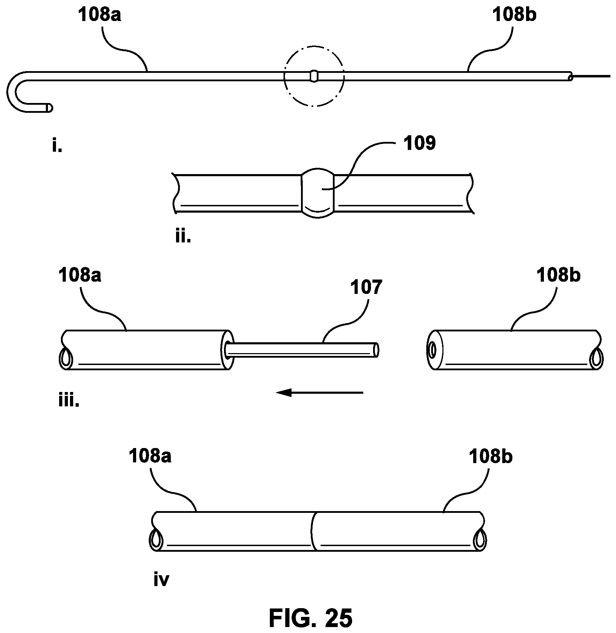

In some embodiments, the shaft of elongate puncture device 100, radiopaque marker 104, and proximal marker 116 have outer diameters<= 0.035" (0.89 mm). Radiopaque marker 104 is comprised of platinum and iridium (Pt/Ir) and has an inner diameter >= 0.01" (0.25 mm). Mandrel 108 of the guidewire is made of Nitinol designed to be kink resistant. An alternative embodiment (Fig. 25) is a composite of super elastic material such as Nitinol to provide a flexible kink resistant distal tip portion 108a and a proximal wire portion 108b of a stiffer alloy such as stainless steel. These materials can be welded (e.g. weld 109), press fit or glued together using an inner mandrel section 107 which extends from distal tip portion 108a in the example of Fig. 25. In further alternative embodiments, inner mandrel section 107 extends from proximal wire portion 108b.

The body of the guidewire (elongate puncture device 100) is insulated with polytetrafluoroethylene (PTFE). While typical embodiments of elongate puncture device 100 have an outer diameter of<= 0.035" (0.89 mm), any size outer diameter of the guidewire is acceptable as long as it fits within the dilator used for an epicardial procedure. Alternative embodiments of radiopaque marker 104, which are components of smaller diameter elongate puncture devices, have an inner diameter smaller than 0.01" (0.25 mm). While a typical embodiment of introducer 130 has an inner diameter of >= 0.035" (0.89 mm), other inner diameter sizes of the introducer are possible so long as the elongate puncture device 100 used in a procedure can pass through.

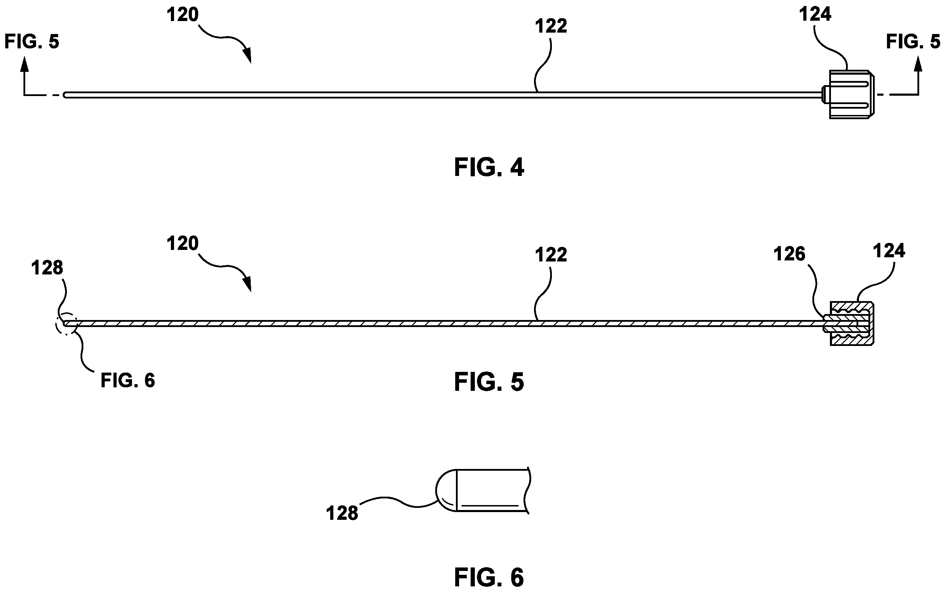

Fig. 4 is an illustration of an embodiment of stylet 120 which includes stylet mandrel 122, stylet cap 124, adhesive 126, and rounded end 128. In alternative embodiments, the tip is sharp. Some alternative embodiments do not include adhesive 126.

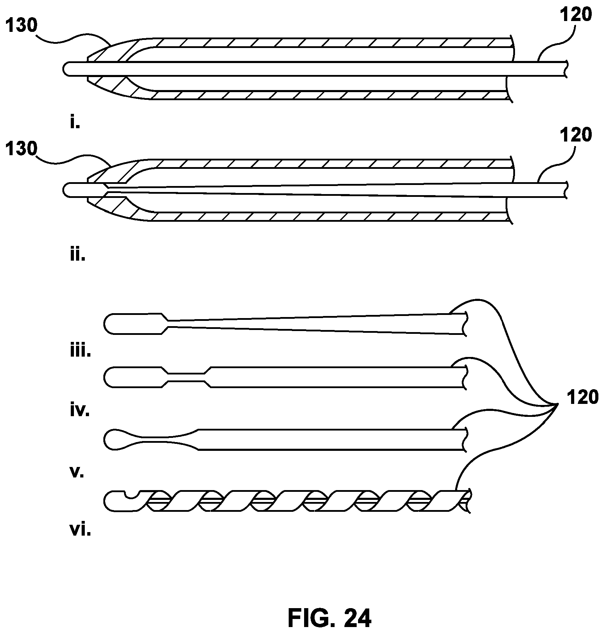

Fig. 5 is a cross sectional view of the embodiment of Fig. 4 which internal details of stylet cap 124. Fig. 6 is an illustration of detail A of Fig. 4 showing rounded end 128. In alternative embodiments (Fig. 24, ii to v) the cross section varies along the length of the stylet 120 necking down (or tapering) towards the distal tip to allow better flow of contrast or fluids through the tip of the introducer when the stylet is installed. Fig. 24i illustrates a non-tapered stylet 120 inside of an introducer 130. Fig. 24vi illustrates a spiral embodiment of stylet 120.

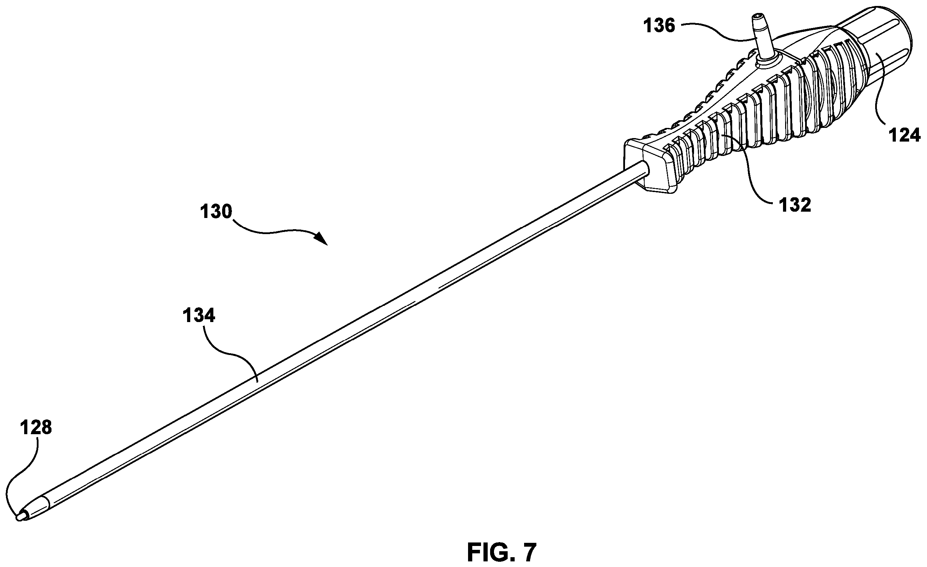

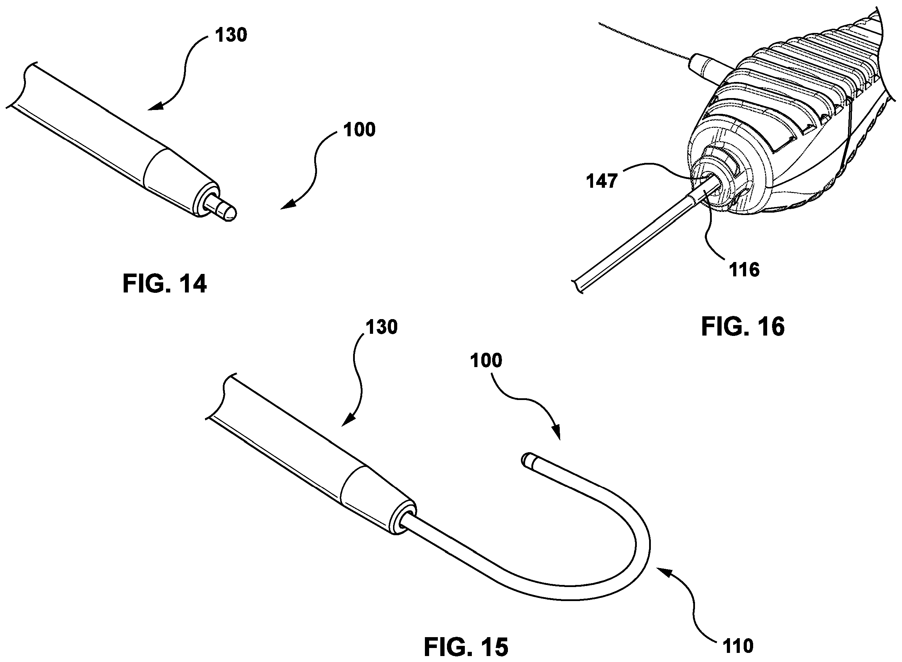

Fig. 7 shows an embodiment of an introducer 130 with a stylet contained therein. Introducer 130 includes an introducer shaft 134 connected to hub 132. Hub 132 has a male side port 136 for connecting to tubing 144 (Fig 13) and a receiving opening (Fig. 16) for receiving a stylet or wire. Alternative embodiments have other side port configurations that are operable to deliver and withdraw fluid. Stylet cap 124 of the installed stylet is proximal of hub 132 and rounded end 128 of the stylet is distal of introducer shaft 134. In some embodiments introducer shaft is a reinforced 8Fr shaft covered with high-density polyethylene (HDPE) comprised of 20% BaS04. The introducer 130 is operable to accommodate a flexible elongate puncture device allowing the introducer 130 (with a elongate puncture device inserted therein): traverse through adipose tissue to reach the target tissue; facilitate the delivery of RF energy by supporting the proper alignment, orientation and position of the distal tip of an elongate puncture device; and allow the guidewire to be deployed into the pericardial space.

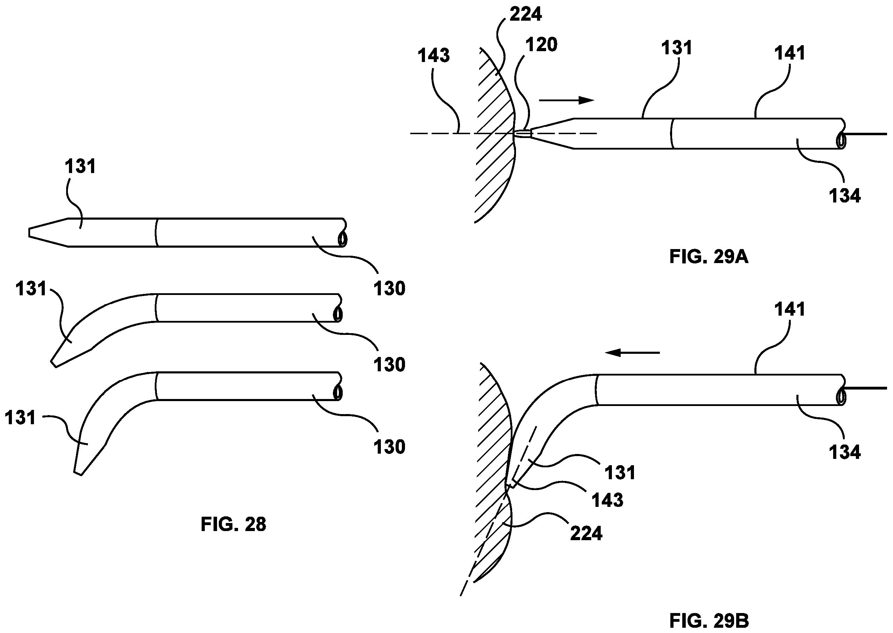

Alternative embodiments of the introducer distal tip 131 may be straight, curved or bent between 15-45 degrees, such as in the examples of Fig. 28. A curved or bent tip would allow the ability to align, orientate and position the distal tip of the elongate puncture device more tangential to the cardiac silhouette.

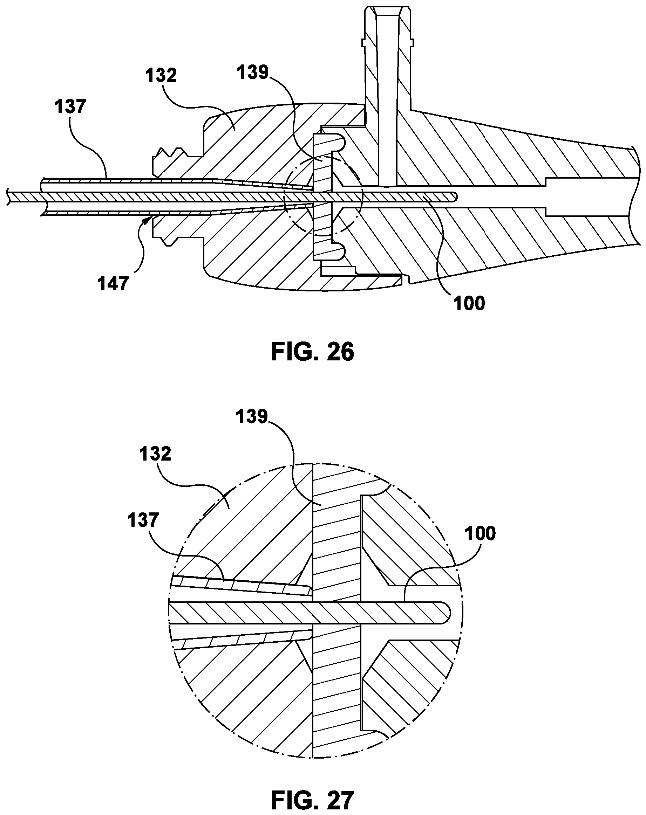

The introducer is design to allow 'front loading', the insertion of the guidewire through the proximal hub and/or 'back loading', through the distal tip of the introducer. Alternative embodiments allow only certain compatible guidewires to limit use. Referring to Fig. 26 and Fig. 27, a tip straightener 137 with a distal tip radius of less than .057" (1.45 mm) allows for insertion of elongate puncture device 100 through receiving opening 147 and valve 139 and into the introducer proximal hub 132 inside diameter. This allows the distal tip of the introducer to gain close approximation to the valve to facilitate seamless guidewire insertion.

Alternative embodiments of the introducer is the ability to accommodate and introduce multiple guidewires for ease of downstream work flow. Introducer can accommodate multiple guidewires up to 0.014 inches or 0.018" (0.36 mm or 0.46 mm) in diameter, for example, if the inner diameter of the introducer is 0.035 inches (0.89 mm).

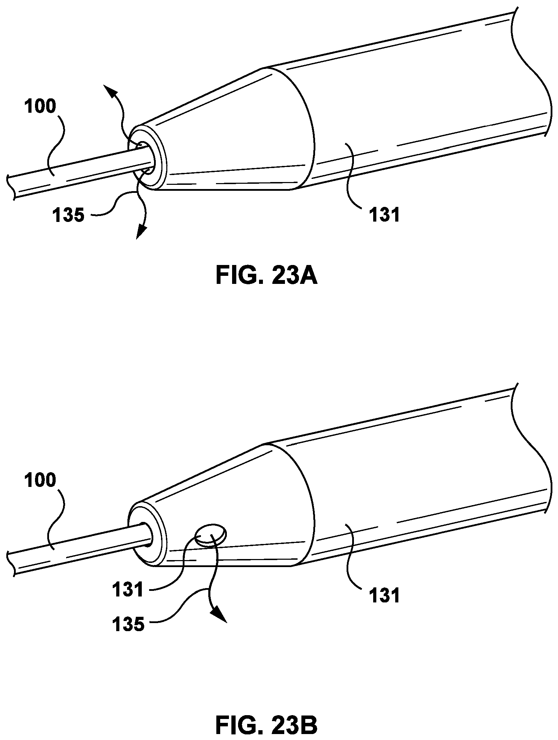

The introducer 130 and elongate puncture device 100 are configured such that introducer 130, even with an inserted elongate puncture device, has the ability to deliver and withdraw fluid while cannulating the elongate puncture device. Introducer shaft 134 is typically >= 5" (12.7 cm). In one embodiment (Fig. 23A), the radial gap between introducer tip 131 ID and the elongate puncture device 100 OD is> .001" (0.025 mm) to provide adequate contrast flow 135 (> 15ml/min at 10 PSI (or 69 Kilo Pascal)) wherein contrast flow 135 is measured by delivering fluid by hand into a beaker for a minute at a constant 10 psi (69 Kilo Pascal) and then measuring the volume of fluid at the end of that minute. In one such embodiment, the introducer tip lumen ID is .0385 (0.978 mm) +/-.0005 inches (0.013 mm), and the maximum elongate puncture device OD is .0350 (0.889 mm) +/- .0005 inches (0.013 mm), whereby the minimum gap with this geometry is .00125" (0.032) mm. Another embodiment (Fig. 23B) of the introducer has side ports 131 located at the introducer tip 131 to allow contrast flow 135. The introducer shaft 134 has a length that is operable to provide adequate stiffness and control to the physician, and to reach the pericardial sac while providing a handle outside of the patient's chest wall. In alternative embodiments, Introducer shaft 134 is less than 5" (12.7 cm). To provide adequate reach and controlled linear trajectory, one embodiment of introducer 130 has a shaft of about 15 cm and a stiffness> 0.04 Nm2 and for a shaft 12 cm a stiffness> .03Nm2. Another alternative length is from 4 to 6 cm to accommodate younger children, and is from 7 to 10 cm for older children.

Alternative embodiments (Fig. 29A and Fig. 29B) of the distal tip 131 of the introducer 130 include a flexible tip 2-3 cm in length. The tip is supported by the rigid distal tip of the assembled stylet 120 (Fig. 29A) to facilitate the ability to traverse through adipose tissue. However, once the stylet is removed, the distal tip of the introducer becomes flexible. A flexible tip allows alignment, orientation and positioning of the elongate puncture device to be more tangential to the cardiac silhouette (Fig. 29B), instead of a perpendicular approach.

An alternative embodiment is a manually reshape-able introducer shaft. This allows the physician to create the desired curve on the shaft of the introducer to facilitate a more curve device insertion trajectory to get underneath the sternum to reach the pericardial sac. This ability reduces the required length of the device in patients with large abdomens or with patients with a more inferior intercostal margin (rib cage).

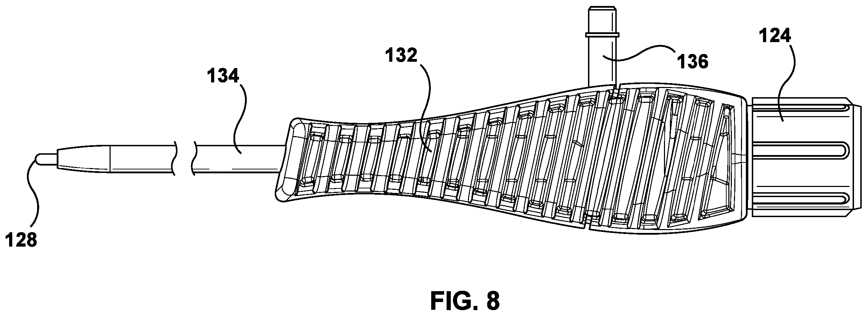

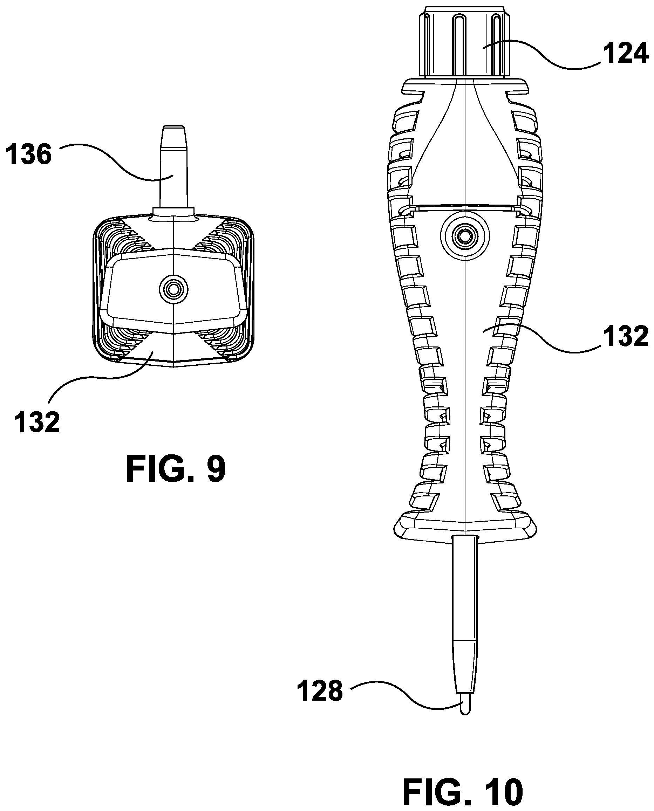

Fig. 8 is a side view of the embodiment of Fig. 7 showing introducer shaft 134, hub 132, and male side port 136 of the introducer, and stylet cap 124 and rounded end 128 of the stylet. When a blunt tipped stylet 120 is introduced into introducer 130, the stylet-introducer combination is operable to be advanced through adipose tissue in order to reach the target tissue. Fig. 9 is an end view of the embodiment of Fig. 7 illustrating hub 132 and male side port 136. Fig. 10 is a top view of the embodiment of Fig. 7 which shows stylet cap 124, hub 132, and rounded end 128 of the stylet distal of introducer.

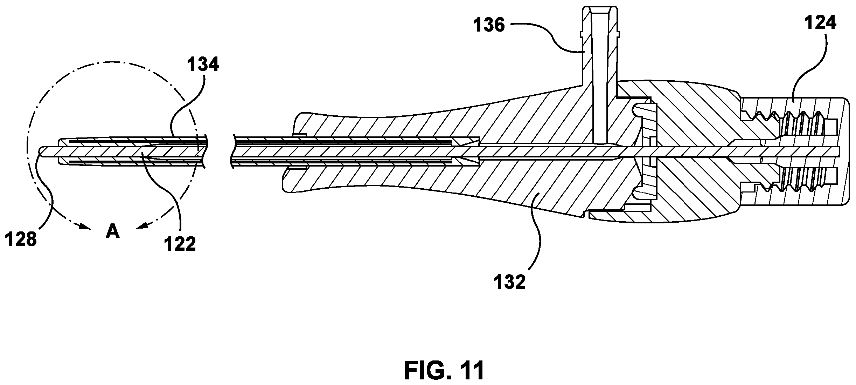

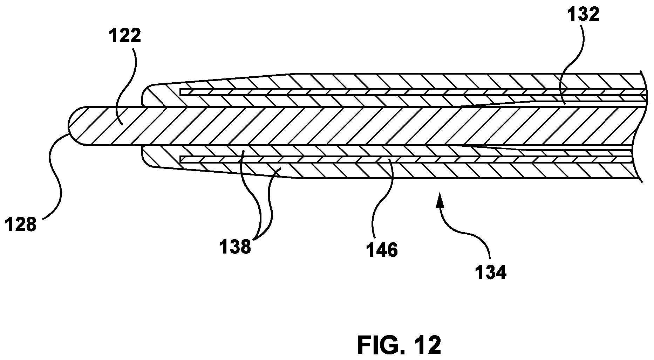

Fig. 11 is a cross sectional view of the embodiment of Fig. 8. Fig. 11 shows stylet mandrel extending from rounded end 128 to inside of stylet cap 124. To provide for advancing through tissue, rounded end 128 of stylet 120 has a diameter of .0375 inches (0.95 mm) or less, while still being less sharp than a Tuohy needle. Fig. 12 is an illustration of detail A of Fig. 11 which illustrates insulation 138 is on the inside of the lumen defines by introducer shaft 134 as well as on the outside surface of introducer shaft 134. In some embodiments, introducer 130 has an inner diameter>= 0.035" (0.89 mm). The shaft of introducer comprises internal metal tube 146 which is sandwiched (or interposed) between two layers of HDPE (insulation 138) with both ends of the layers of insulation being tipped (i.e. joined together to form a tip) to bond the layers. An introducer 130 with such a configuration can be combined with an elongate puncture device 100 which is a wire is insulated most of its length with the exceptions of the electrode tip and proximal connector to provide apparatus which is effective collecting local EGM. An example is a kit comprising an introducer 130 and an elongate puncture device 100, the elongate puncture device including a distal tip electrode 102 which enables recording epicardial EGM and a shaft of the elongate puncture device is electrically insulated to reduce noise in any collected local EGM, with the introducer comprising an introducer shaft 134 which connected to a hub 132, the introducer shaft comprising an internal metal tube 146 which is interposed between two layers of electrical insulation 138 with both ends of the two layers being joined together to bond the layers to reduce noise in any EGM collected by the elongate puncture device.

The insulation at distal end of introducer 130 is shaped to form a tip which enables transition through tissue when the intruder is advanced. The length of the introducer shaft 134 is >= 5" (12.7 cm). Hub 132 is comprised of plastic and includes a silicone seated valve. Fig 12 also illustrates the space between stylet mandrel 122 and introducer shaft 134 decreases toward the distal end of introducer shaft 134 i.e. there is a tighter fit between stylet mandrel 122 and introducer shaft 134 at the distal end of introducer shaft 134.

Fig 13 is an illustration of apparatus of an embodiment which shows the size of the components relative to each other. Fig. 14 illustrates an elongate puncture device 100 extending from introducer 130. Only part of straight portion 112 of the guidewire (Fig. 1) is protruding from the introducer. In some embodiments, the tip of the elongate puncture device 100 is only protruding out 2 mm from the blunt tip of the introducer. This can act as a depth stop in limiting how deep the tip of the elongate puncture device can penetrate into the targeted tissue. The tip protrusion length could change depending on support of the tip by the introducer and electrode size. Fig. 15 illustrates a situation in which a distal end portion 110, which has a J-profile, is extended from an introducer 130.

Fig. 16 shows the proximal end of an introducer hub wherein an elongate puncture device has been inserted such that proximal marker 116 of the guidewire is lined up with the proximal edge of the hub. Proximal marker 116 is visible to a user without the use of an imaging system i.e. it is visible by the naked eye. While typical embodiments of proximal marker 116 are visual markers, in some alternative embodiments, the proximal marker 116 is a tactile marker. In typical embodiments of the apparatus, this positioning of proximal marker 116 indicates the electrode 102 (Fig 1) is outside the introducer close to the distal end of the introducer i.e. electrode 102 is deployed by positioning it slightly extended out of the distal end of the introducer.

Fig. 17 is a diagrammatic cross sectional view of an introducer 130 with an elongate puncture device 100 installed therein. In typical embodiments, both the introducer 130 and elongate puncture device 100 include markers for positioning the elongate puncture device 100 relative to introducer 130. In the embodiment of Fig. 17, introducer shaft 134 has a distal marker 142 at its distal end for indicating the position of the distal end of introducer shaft 134 under imaging, and the elongate puncture device has a radiopaque marker 104 at its distal end for indicating the position of the distal end of elongate puncture device under imaging. The position of the distal ends relative to each other can also be determined under imaging. Fig 17 also shows the elongate puncture device having proximal marker 116 at the proximal end of the introducer hub. In this illustrated embodiment, the tip of the elongate puncture device is extending out of the introducer shaft when proximal marker 116 is positioned at the proximal end of the introducer hub e.g. the line marker on the elongate puncture device helps to inform when the tip is deployed. In alternative embodiments, the tip of the elongate puncture device is line up with the distal tip of the introducer shaft when the proximal marker 116 is positioned at the proximal end of the introducer hub.

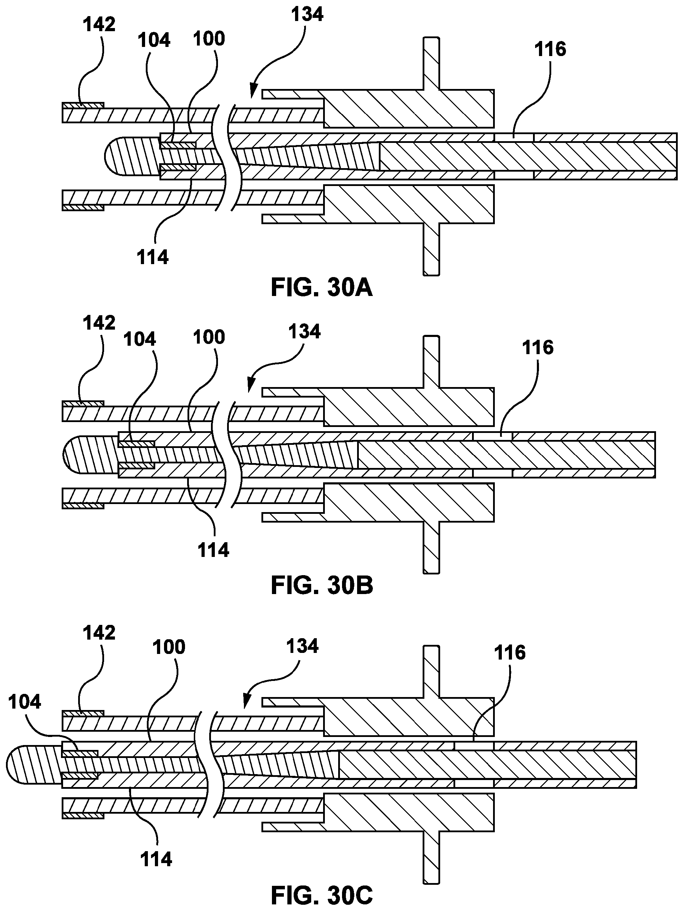

In the embodiment of Figs. 30A to 30C, introducer shaft 134 has a distal marker 142 at its distal end for indicating the position of the distal end of introducer shaft 134 under imaging, and the elongate puncture device has a radiopaque marker 104 at its distal end for indicating the position of the distal end of elongate puncture device 100 under imaging. Figs. 30A to 30C show the steps of a method of advancing an elongate puncture device 100 through the introducer shaft 134. Fig. 30A shows the elongate puncture device 100 is positioned to have the distal end of proximal marker 116 at the proximal end of the introducer hub while the tip of the tip of the elongate puncture device still inside of the lumen of the introducer shaft. The elongate puncture device is advanced to the configuration of Fig. 30B wherein the middle of proximal marker 116 at the proximal end of the introducer hub and the tip of the tip of the elongate puncture device lines up with the tip of the introducer shaft 134. The elongate puncture device is further advanced to the configuration of Fig. 30C wherein the proximal end of proximal marker 116 is at the proximal end of the introducer hub and the tip of the elongate puncture device extends beyond the tip of the introducer shaft 134. The configuration on Fig. 30C further includes distal marker 142 of introducer shaft 134 lining up with radiopaque marker 104 at of the distal end of introducer shaft 134, which under imaging, would confirm the relative positioning of the elongate puncture device and introducer.

In some examples of the method, the user positions the elongate puncture device relative to the introducer using the proximal marker 116 without using an imaging system such a fluoroscopy in a step that can be called, 'macro-positioning'. Subsequent to the 'macro-positioning', the user turns on an imaging system (e.g. fluoroscopy) for more precise positioning of the elongate puncture device relative to the introducer and the target tissue in a step that can be called micro-positioning. By using the proximal and distal markers, a user can perform the early part of positioning the apparatus without fluoroscopy to thereby reduce the amount of X-rays the user and patient are exposed to when compared to performing the entire procedure under fluoroscopy. In some alternative embodiments of the method, the part of the procedure involved with positioning the elongate puncture device relative to the introducer is performed without any fluoroscopy.

Fig. 18 is an illustration of the layers surrounding a heart. Ventricle 230 is surrounded by the muscular tissue of the heart, myocardium 228. Pericardium 226 surrounds the myocardium 228 and is comprised of fibrous pericardium 232, parietal layer 234, pericardial cavity 236, and epicardium 238. The pericardial cavity is often referred to as the pericardial space or the space.

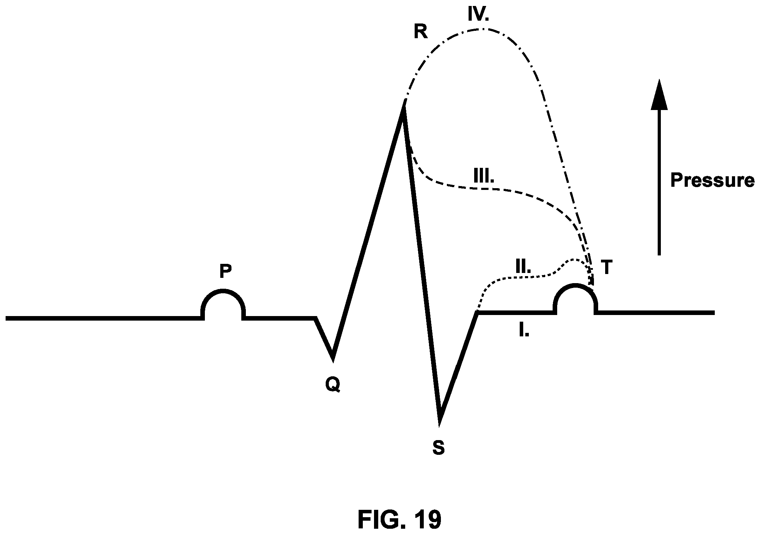

Fig. 19 is an illustration of epicardial EGM waves showing elevated ST segments in response to tenting the pericardium resulting in an ischemic response of the tissue affecting the local epicardial EGM. Such waves can be collected using electrode 102 of an elongate puncture device 100 when the elongate puncture device is advanced and pushed against the pericardium. Line (I) of Fig. 19 is the wave for no tenting (or insignificant pressure) being applied on the pericardium and line (IV) is the wave for very significant tenting of the pericardium. Lines (II) and (III) are for between the amounts of tenting resulting in lines (I) and (IV).

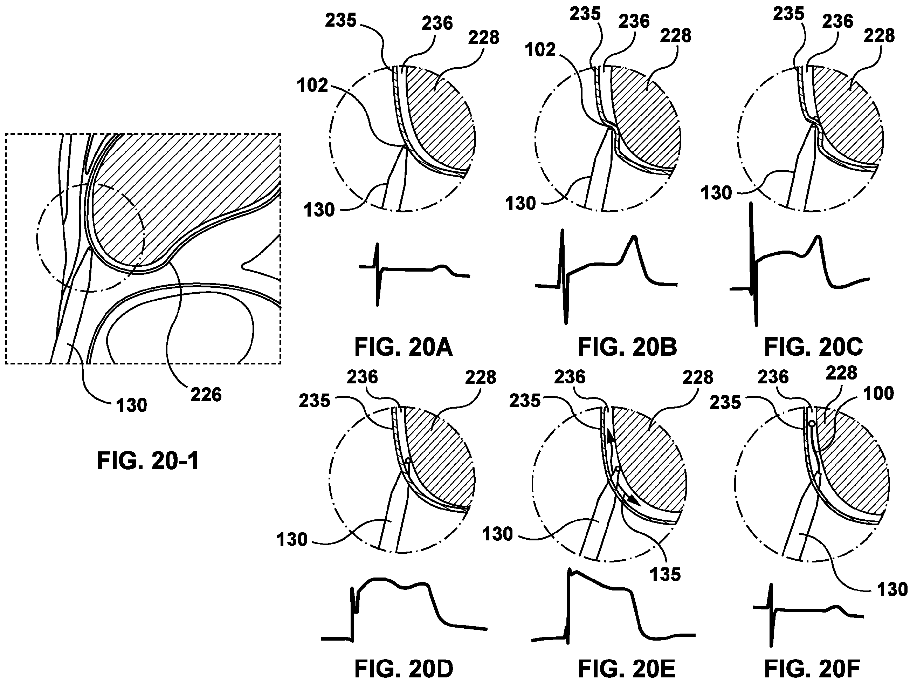

Fig. 20-1, and Figs. 20A to Fig. 20F illustrate a method. The following described method can be performed using the different embodiments of the apparatus previously described. Fig. 20-1 illustrates the sub-xiphoid approach of introducer 130 relative to pericardium 226 and the surrounding anatomy. When a blunt tipped stylet 120 is introduced into introducer 130, the stylet-introducer combination is operable to be advanced through adipose tissue in order to reach the target tissue. Typically, stylet 120 is fastened to the proximal end of the hub 132 of introducer 130. A physician makes a small nick in the patient's skin to allow the introducer 130 with the protruding stylet tip to pass through the dermal and adipose layers. The blunt tip of stylet 120 provides the necessary cutting action to separate tissue to reach the target. Using a stylet with a blunt tip is advantageous over using stylet with a sharp beveled tip that will lacerate the tissue as the device traverses through tissue. A blunt tip is also more forgiving if inadvertently advanced too close to the target tissue. It is less likely to puncture the epicardial surface of the heart. Furthermore, other factors being equal, a stylet with a blunt tip provides more tactile feedback than a stylet with a sharp tip. Note that the procedure is done under fluoroscopy.

After the stylet 120 is removed from introducer 130, introducer 130 has a rigid shaft that can support the deployment of an elongate puncture device 100 which is flexible and 10 has a soft tip. The guidewire has a dome tip (electrode dome 103) that can dock with the tissue preventing premature puncture. The physician is able to optimize their position of the tip of the wire. The introducer has an internal metal tube 146 with a lip that is typically within 0.5 to 2 mm from the tip. The internal metal tube 146 functions as a radiopaque marker to indicate where the tip of introducer 130 is located. The physician places the tip of the elongate puncture device 100 15 outside the introducer 130 to ensure proper delivery of RF electrical energy. The physician can advance and retract the tip of the wire under fluoroscopy to gauge how far the wire is sticking out from the tip of the introducer. Fig. 20A illustrates electrode 102 of the elongate puncture device being in contact with the pericardium without any significant force being exerted. The associated EGM, with a low ST segment, is shown below (in the lower part of the figure). Fig. 20B shows the introducer 130 and electrode 102 of the guidewire exerting some force to cause tenting of the pericardium. The wave associated with Fig. 20B is higher than the wave associated with Fig. 20A. In this method, the EGM can be measured while moving devices, or before or after devices are moved.

Fig. 20C illustrates energy being delivered through the electrode to the tissue of the pericardium. The ST segment of the EGM wave shows higher elevation than that of the previous waves. The guidewire has a blunt electrode at the tip to safely dock with tissue and thereby facilitate directly delivering RF energy to the targeted tissue, which the electrode 102 is in contact with. A short pulse (e.g. 1/3 second) of high voltage AC is delivered by the electrode to create a hole in the pericardial sac 235, after which the sac will prolapse over the guidewire tip. The delivery of a short pulse (e.g. 1/3 second) of RF energy aids in limiting depth of puncture. A physician could use a longer pulse but risk the chance of penetrating deep into the myocardial tissue. In alternative embodiments the pulse is shorter or longer depending on power settings and required tissue penetration.

Fig. 20D shows the distal portion of introducer after being advanced into the pericardial cavity 236 and the associated wave, which has an elevated ST segment. Introducer 130 has valve 140 (e.g. a stopcock) attached to it by tubing 144 (Fig 13) and a valve built into hub 132 (a handle) to allow concurrent delivery of contrast media. This will allow the physician to confirm the tip location of introducer 130 with or without the guidewire in place. The inner diameter of the introducer 130 is large enough to accommodate contrast media flow with a cannulated wire. This configuration of the devices allows the physician to withdraw fluid from the pericardial space. For example, if there is excess fluid or blood, it can be aspirated. The arrows in Fig. 20E represent imaging fluid being injected into pericardial cavity 236. If the 10 imaging of the fluid indicates the fluid is in the pericardial cavity 236, then access to the cavity has been achieved.

Fig. 20F shows a tip the elongate puncture device 100 being advanced into the pericardial cavity 236. The guidewire (elongate puncture device 100) can then be advanced more fully into the space. If the tip of the elongate puncture device continues to move freely after accessing the pericardial space, a physician can confirm access by wrapping the guidewire around the heart at least once (i.e. advancing around the cardiac silhouette) and visualizing under fluoroscopy that the guidewire outlines the cardiac silhouette. The distal 3 cm of the elongate puncture device 100 has a radiopaque coil 106 which increases visibility of the distal end portion 110 under visualization, thereby helping to confirm access to the pericardial space when advancing the tip of the elongate puncture device.

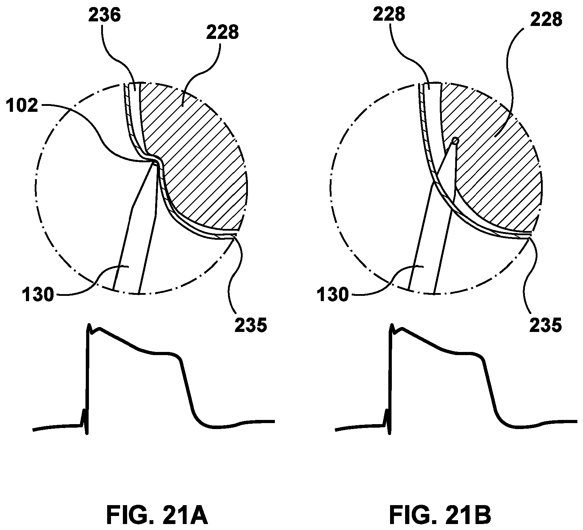

The tip of the wire starting to bunch up when the physician is attempting to advance the elongate puncture device can indicate access to the pericardial cavity 236 has not been achieved. Fig. 21A illustrates the complication scenario in which the epicardium is not punctured and significant tenting occurs. The significant tenting is caused by a significant pressure being applied, resulting in a wave with an elevated ST segment. Fig. 21B illustrates another complication scenario an elongate puncture device passes through the pericardial cavity 236 and into the myocardium 228, resulting a in a wave with an elevated ST segment which is similar to the wave of Fig. 21A.

Elongate puncture device 100 has features facilitating epicardial EGM collection (and non-epicardial EGM collection e.g. EGM collection at the septum of a heart).

Typical embodiments of elongate puncture device 100 comprise a single electrode at the tip of the guidewire which enables recording epicardial EGM from distal tip of the elongate puncture device 100 while tenting tissue. Alternative embodiments of elongate puncture device 100 have multiple electrodes at the tip to collect additional information.

Typical embodiments of elongate puncture device 100 are comprised of material which is electrically conductive to allow for the flow of current.

The electrode 102 is comprised of material which provides for a stable impedance contact with tissue. Impedance depends on the electrode material, tissue, electrode surface area 5 and the temperature. A lower impedance means it less likely for EGM signal to go silent (a DC offset problem). In typical embodiments the electrode is comprised of a solid material. In alternative embodiments, the electrode has a coating of electrically conductive material.

If the electrode surface area is too large, EGM recording resolution will be negatively impacted. An electrode surface area which is too small is susceptible to electrical interference. Some embodiments of the electrode have hemispherical electrode tips (electrode dome 103) with outer diameters between 0.024" (0.62 mm) and 0.035" (0.89 mm). Such embodiments were tested on in vivo porcine study which found that the effects of varying the OD and surface area of the electrode tip (with a CardioLab electrophysiology recording system) showed no observable differences between hemispherical electrode tips with outer diameters between 0.024" (0.62 mm) and 0.035" (0.89 mm). Electrode dome 103 has a dome geometry which ensures uniform contact with tissue at various angles of trajectory. To enable the user to collect epicardial EGM to provide guidewire tip location, on embodiment of the tip electrode has a dome OD > .027" (0.686 mm) and a wire tip surface area of about 1.8 to about 2.4 mm2 (wherein the dimensions are accurate to one decimal place) such that the electrode is small enough for puncturing tissue while being large enough for effective EGM collection. Further, to create a puncture smaller than al 7Ga Tuohy needle (OD <.058" or 1.5 mm), some embodiments have a distal tip electrode OD of about .0275 inches (0.70 mm).

The shaft of elongate puncture device 100 has features which facilitate collecting an EGM signal. The shaft of elongate puncture device 100 is electrically insulated to reduce noise from the collected local EGM. Also, the inner diameter of introducer 130 and the outer diameter of elongate puncture device 100 enable the introducer and elongate puncture device to fit together with clearance to facilitate delivering contrast agent at the same time as collecting epicardial EGM.

The following method can be performed using the different embodiments of the apparatus previously described. A method which uses the above described devices to puncture a target tissue to gain access to the pericardial cavity 236 comprises the steps of:
(1) Connecting an electrically insulated wire, with one distal pole (e.g. an elongate puncture device 100 which is a RF guidewire ), to a recording system that has an electrocardiographic reference to the patient (e.g. EKG pads, subcutaneous reference or intracardiac reference catheter).
(2) Puncturing the patient's skin in a minimally invasive manner via the subxiphoid, parasternal intercostal or apical approach with a rigid introducer and an engaged stylet. A small nick is made in the patient's skin below the xiphoid process to initiate the introduction.
(3) The rigid introducer 130 and stylet 120 are advanced traversing through various dermal layers and toward the pericardial sac of the heart under the guidance of fluoroscopy and tactile feedback to position the tip of the introducer near the pericardial sac. In some embodiments of the method, the sty let is insulated and connected to a recording system to facilitate collecting electrocardiographic information to help in positioning the tips of the devices when approaching the heart (i.e. in large scale positioning).
(4) The stylet 120 is removed, with the blunt radial tip of the introducer docking with the pericardial sac, and replaced with the elongate puncture device 100.
(5) Elongate puncture device 100 is advanced with the introducer 130 contacting the outside of the pericardial sac.
   (5a) Local epicardial electrograms, EGMs are recorded from the electrode tip of elongate puncture device 100. As the guidewire travels inside introducer 130 there is little to no signal. Once the tip of elongate puncture device 100 is near or protruding from the tip of introducer 130, the guidewire will start to collect an EGM signal. Under fluoroscopic guidance, the guidewire is advanced until the tip is protruding 2mm from the tip of the introducer, thereby making contact with the pericardial sac. The tip of elongate puncture device 100 is blunt to prevent lacerating the hearts surface or prematurely puncturing the pericardial sac or myocardial tissue. The blunt tip allows the physician to optimize the location, orientation and angle of approach of the device.
   (5b) As the tip of the elongate puncture device contacts the pericardial sac, the amplitude of the waveforms will increase with higher mechanical force (tenting) against the heart due to transient ischemic response of the pericardial sac and epicardial tissue. Note: if the device (electrode 102) is not up against the pericardial sac it will collect far field EGMs or other local EGMs, for example, from the diaphragm.
   (5c) Elongate puncture device 100 is docked with the introducer 130, and the elongate puncture device and the introducer are advanced or retracted together to increase or decrease force required for a successful puncture. The local EGM acts as an indirect pressure sensor. The recorded EGM trace will show evidence of an elevated ST segment. The physician can monitor the amount of tenting of the tissue indicated by the amplitude of the ST segment. As part of an interactive approach, a set elevated ST segment is selected which the physician believes to provide adequate tenting to avoid overshoot. The physician may also use tactile feedback when grasping the guidewire when docked against the pericardial sac. Contrast medium can also be injected through the introducer exiting at the tip to confirm adequate tenting of the sac.
(6) Delivering energy to the pericardial sac through the electrode 102 at the tip of elongate puncture device 100. For safety, energy is typically delivered as a pulse (e.g. 1/3 second). Some embodiments include delivering less energy in the pulse by selecting a lower power level or shorter pulse time if a higher pressure level is selected in step (5c). In such embodiments, there is an inverse proportional relationship between the pressure exerted on the sac and the amount of energy delivered in a pulse. In typical embodiments, the physician starts with a low pressure level in the first iteration to avoid overshoot, and the amount of pressure exerted on the pericardial sac is increased with each iteration (while maintaining the same pulse time and energy level) with the physician looking for higher ST elevation to reattempt access.
   (6a) Checking if the pericardial sac has been punctured and access to the pericardial cavity has been gained (using previously described techniques). Upon puncture, the pericardial sack will then prolapse over the guidewire tip. If access has not been gained the elongate puncture device is retracted and repositioned on the pericardial sac for another attempt, and the physician returns to step (5c).
(7) Monitoring the electrical activity of the epicardial surface using electrode 102 on the distal tip of elongate puncture device 100 (after RF puncture of the pericardial sac provides access to the pericardial cavity).
(8) Deploying the elongate puncture device 100 further into the pericardial space. Deploying the elongate puncture device 100 further into the pericardial space will relax pressure at the tip and cause a change in the EGM trace. The physician may deliver contrast medium to further confirm access.

Some embodiments include the further step (9) of deploying the guidewire for tracking along the epicardial surface of the heart while collecting local epicardial EGM. The recorded signal can be inputted into EP mapping systems for added information.

Elongate puncture device 100 can support a sheath. Typical embodiments include the further step of advancing a sheath and dilator over elongate puncture device 100. Once the sheath tip is positioned appropriately within the pericardial space, the dilator and guidewire are removed. Subsequently, the sheath provides an access portal for advancement and placement for devices such as mapping or ablation catheters to facilitate diagnosis or therapy of a variety of arrhythmias. Some such embodiments of the method include the use a steerable sheath.

Also, some embodiments use a system including an amplifier with the ability to amplify voltages and measure impedance from an electrode across a range of anatomically relevant frequencies (from about 10kHz to about 80kHz).

While not considered as a step in the prescribed method, it is possible for the elongate puncture device to be embedded into the epicardium or ventricle after RF puncture. In these situations, the amplitude of the electrical activity (ST segment) decreases, providing feedback to the user that the guidewire should be retracted and RF puncture could be reattempted. These situations can also be confirmed with fluoroscopy.

The above described methods can use different embodiments of the above described embodiments of apparatus (i.e. different embodiments of devices).

The embodiments of the invention described above are intended to be exemplary only. The scope of the invention is therefore intended to be limited solely by the scope of the appended claims.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

## Claims

1. An elongate puncture device (100) comprising: a mandrel (108) which is electrically conductive and covered by a layer of insulation (114),
wherein the layer stopping short of a distal end of the mandrel (108) such that the distal end of the mandrel (108) is electrically exposed to define a distal tip electrode (102), a portion of the mandrel (108) being surrounded by a visible marker (117), the visible marker (117) being covered by the layer, **characterized in that** the layer is a clear layer and the portions of the elongate puncture device (100) at and adjacent the visible marker (117) have a constant outer diameter.

2. The elongate puncture device (100) of claim 1, wherein the mandrel (108) is surrounded by an oxide coating (118) which is covered by the clear layer of insulation (114), wherein for at least one portion of the mandrel (108) the oxide coating (118) has been removed such that said at least one portion defines at least one visible marker (117).

3. The elongate puncture device (100) of claim 1, wherein the clear layer comprises a heat-shrink layer (115) wherein the heat-shrink layer (115) comprises a polytetrafluoroethylene material.

4. The elongate puncture device (100) of claim 2, wherein the mandrel (108) is comprised of a nitinol and the oxide coating (108) is comprised of a layer of titanium dioxide.

5. The elongate puncture device (100) of any one of claims 1 to 4, wherein the elongate puncture device (100) is flexible.

6. The elongate puncture device (100) of any one of claims 1 to 5, wherein the mandrel (108) is electrically conductive, and a proximal end portion of the mandrel (108) is uninsulated and operable for connecting to a power supply such that the distal tip electrode (102) is in electrical communication with the power supply, and energy can be delivered through the distal tip electrode (102) to tissue, and the distal tip electrode (102) enables recording epicardial EGM.

7. The elongate puncture device (100) of claim 6, wherein a shaft of the elongate puncture device (100) is electrically insulated to reduce noise from any collected local EGM.

8. The elongate puncture device (100) of any one of claims 1 to 7, further comprising a distal end portion (110) which has a J-profile, and a radiopaque coil (106) which extends around a curve of the distal end portion (110) wherein an end of the radiopaque coil (106) can be used as a distal tip marker, and the radiopaque coil (106) has echogenic properties when using ultrasound to enable visualization of a tip of the elongate puncture device (100).

9. The elongate puncture device (100) of any one of claims 1 to 8, wherein the elongate puncture device (100) comprises a radiofrequency guidewire.

## Patentansprüche

1. Längliche Punktiervorrichtung (100), umfassend: einen Dorn (108), der elektrisch leitend ist und von einer Schicht (114) von Isolation bedeckt ist,
wobei die Schicht kurz vor einem distalen Ende des Dorns (108) aufhört, so dass das distale Ende des Dorns (108) elektrisch exponiert ist, um eine distale Spitzenelektrode (102) zu definieren, wobei ein Teil des Dorns (108) von einer sichtbaren Markierung (117) umgeben ist, wobei die sichtbare Markierung (117) von der Schicht bedeckt ist, **dadurch gekennzeichnet, dass** die Schicht eine klare Schicht ist und die Teile der länglichen Punktiervorrichtung (100) an und neben der sichtbaren Markierung (117) einen konstanten Außendurchmesser aufweisen.

2. Längliche Punktiervorrichtung (100) nach Anspruch 1, wobei der Dorn (108) von einer Oxidbeschichtung (118) umgeben ist, die von der klaren Schicht (114) aus Isoliermaterial bedeckt ist, wobei für mindestens einen Teil des Dorns (108) die Oxidbeschichtung (118) entfernt wurde, so dass der mindestens eine Teil mindestens eine sichtbare Markierung (117) definiert.

3. Längliche Punktiervorrichtung (100) nach Anspruch 1, wobei die klare Schicht eine Wärmeschrumpfschicht (115) umfasst, wobei die Wärmeschrumpfschicht (115) ein Polytetrafluorethylenmaterial umfasst.

4. Längliche Punktiervorrichtung (100) nach Anspruch 2, wobei der Dorn (108) aus einem Nitinol gebildet ist und die Oxidbeschichtung (108) aus einer Titandioxidschicht gebildet ist.

5. Längliche Punktiervorrichtung (100) nach einem der Ansprüche 1 bis 4, wobei die längliche Punktiervorrichtung (100) flexibel ist.

6. Längliche Punktiervorrichtung (100) nach einem der Ansprüche 1 bis 5, wobei der Dorn (108) elektrisch leitend ist und ein proximaler Endteil des Dorns (108) nicht isoliert ist und für einen Anschluss an eine Leistungsquelle betätigbar ist, so dass die distale Spitzenelektrode (102) mit der Leistungsquelle in elektrischer Verbindung steht und Energie durch die distale Spitzenelektrode (102) hindurch an Gewebe abgegeben werden kann und die distale Spitzenelektrode (102) eine Aufzeichnung eines epikardialen EGM ermöglicht.

7. Längliche Punktiervorrichtung (100) nach Anspruch 6, wobei ein Schaft der länglichen Punktiervorrichtung (100) elektrisch isoliert ist, um ein Rauschen von jeglichen erfassten lokalen EGMs zu reduzieren.

8. Längliche Punktiervorrichtung (100) nach einem der Ansprüche 1 bis 7, ferner einen distalen Endteil (110) mit einem J-Profil und eine um eine Kurve des distalen Endteils (110) verlaufende strahlenundurchlässige Spule (106) umfassend, wobei ein Ende der strahlenundurchlässigen Spule (106) als distale Spitzenmarkierung verwendet werden kann und die strahlenundurchlässige Spule (106) echogene Eigenschaften aufweist, wenn Ultraschall verwendet wird, um eine Visualisierung einer Spitze der länglichen Punktiervorrichtung (100) zu ermöglichen.

9. Längliche Punktiervorrichtung (100) nach einem der Ansprüche 1 bis 8, wobei die längliche Punktiervorrichtung (100) einen Funkfrequenz-Führungsdraht umfasst.

## Revendications

1. Dispositif de perforation allongé (100) comprenant : un mandrin (108) qui est électriquement conducteur et qui est recouvert par une couche d'isolant (114),
dans lequel la couche est arrêtée à proximité d'une extrémité distale du mandrin (108) de telle sorte que l'extrémité distale du mandrin (108) soit exposée électriquement afin de définir une électrode à pointe distale (102), une partie du mandrin (108) étant entourée par un marqueur visible (117), le marqueur visible (117) étant recouvert par la couche d'isolant, **caractérisé en ce que** la couche est une couche transparente et les parties du dispositif de perforation allongé (100) au niveau du marqueur visible (117) et en une position adjacente à celui-ci présentent un diamètre externe constant.

2. Dispositif de perforation allongé (100) selon la revendication 1, dans lequel le mandrin (108) est entouré par un revêtement en oxyde (118) qui est recouvert par la couche d'isolant (114) transparente et dans lequel, sur au moins une partie du mandrin (108), le revêtement en oxyde (118) a été enlevé de telle sorte que ladite au moins une partie définisse au moins un marqueur visible (117).

3. Dispositif de perforation allongé (100) selon la revendication 1, dans lequel la couche transparente comprend une couche thermo-rétractable (115) et dans lequel la couche thermo-rétractable (115) comprend un matériau à base de polytétrafluoroéthylène.

4. Dispositif de perforation allongé (100) selon la revendication 2, dans lequel le mandrin (108) est constitué par un nitinol et le revêtement en oxyde (118) est constitué par une couche de dioxyde de titane.

5. Dispositif de perforation allongé (100) selon l'une quelconque des revendications 1 à 4, dans lequel le dispositif de perforation allongé (100) est souple.

6. Dispositif de perforation allongé (100) selon l'une quelconque des revendications 1 à 5, dans lequel le mandrin (108) est électriquement conducteur, et une partie d'extrémité proximale du mandrin (108) est non isolée et peut être rendue opérationnelle pour être connectée à une alimentation électrique de telle sorte que l'électrode à pointe distale (102) soit en communication électrique avec l'alimentation électrique, que de l'énergie électrique puisse être délivrée par l'intermédiaire de l'électrode à pointe distale (102) au tissu et que l'électrode à pointe distale (102) permette l'enregistrement d'électrocardiogrammes (EGM) épicardiques.

7. Dispositif de perforation allongé (100) selon la revendication 6, dans lequel un arbre du dispositif de perforation allongé (100) est isolé électriquement afin de réduire le bruit en provenance d'un quelconque EGM local collecté.

8. Dispositif de perforation allongé (100) selon l'une quelconque des revendications 1 à 7, comprenant en outre une partie d'extrémité distale (110) qui présente un profil en J et une bobine radio-opaque (106) qui est étendue autour d'une courbe de la partie d'extrémité distale (110), dans lequel une extrémité de la bobine radio-opaque (106) peut être utilisée en tant que marqueur de pointe distale et la bobine radio-opaque (106) présente des propriétés échogènes lors de l'utilisation d'ultrasons pour permettre la visualisation d'une pointe du dispositif de perforation allongé (100).

9. Dispositif de perforation allongé (100) selon l'une quelconque des revendications 1 à 8, dans lequel le dispositif de perforation allongé (100) comprend un fil-guide à radiofréquences.
